# NOUVEAU FASCICULE DE BREVET EUROPEEN

(11) **EP 1 187 578 B2**
(45) Date de publication et mention de la décision concernant l'opposition: **07.09.2016**
(45) Mention de la délivrance du brevet: 19.08.2009
(21) Numéro de dépôt: 00949527.6
(22) Date de dépôt: 13.06.2000
(51) Int. Cl.: A61F 2/06, A61B 17/22

(54) **KIT POUR LE RETRAIT D'UN FILTRE POUR VAISSEAUX SANGUINS**
KIT ZUM ENTFERNEN EINES BLUTGEFÄSSFILTERS
KIT FOR REMOVING A BLOOD VESSEL FILTER

(30) Priorité: 14.06.1999 FR 9907690
(43) Date de publication de la demande: 20.03.2002
(73) Titulaire: ALN, 20240 Ghisonaccia (FR)
(72) Inventeur: NIGON, Alain, F-83230 Bormes les Mimosas (FR)
(74) Mandataire: Cabinet Plasseraud
(86) Numéro de dépôt international: PCT/FR2000/001624
(87) Numéro de publication internationale: WO 2000/076422

(56) Documents cités:
- EP-A- 0 813 842
- EP-A2- 0 702 936
- EP-B1- 0 518 839
- EP-B1- 0 759 827
- EP-B1- 1 115 451
- WO-A-00/16845
- WO-A-98/16274
- WO-A-98/33443
- WO-A1-98/23322
- US-A- 4 471 777
- US-A- 4 494 531
- US-A- 4 655 219
- US-A- 4 817 600
- US-A- 4 832 055
- US-A- 4 969 891
- US-A- 4 994 079
- US-A- 5 217 484
- US-A- 5 370 657
- US-A- 5 413 586
- US-A- 5 464 408
- US-A- 5 512 037
- US-A- 5 558 652
- US-A- 5 700 269
- G. P. TEITELBAUME ET AL.: 'Insertion and Recovery of a New Retrievable Vena Caval Filter' pages 527 - 533

## Description

La présente invention concerne un kit pour le retrait d'un filtre pour vaisseaux sanguins.

Actuellement, on connaît deux grands types de filtres pour veine cave inférieure. Rappelons qu'un filtre pour veine cave inférieure est un dispositif constitué de brins métalliques ressemblant à une armature de parapluie à moitié ouvert mais constitué uniquement des baleines et de la noix de celui-ci, que l'on installe dans la veine cave inférieure. Les brins métalliques sont munis de crochets à leur extrémité libre, ce qui leur permet de se cramponner à la paroi vasculaire.

Ces brins métalliques sont fins et souples et de ce fait battent en cadence avec les mouvements péristaltiques de la veine cave inférieure. Ces mouvements qui conduisent les brins métalliques à se rapprocher et s'écarter alternativement permettent, au cas où le filtre parapluie bloque un caillot, de le cisailler progressivement et le portionner en petits morceaux qui ne peuvent plus être nuisibles.

Le premier type de filtre est le type définitif : c'est ainsi que l'on installe un filtre parapluie ci-dessus dans la veine cave inférieure, et on l'y laisse définitivement. Le corps médical est toujours réticent à laisser définitivement dans l'organisme humain un corps étranger.

On connaît également les filtres temporaires. Pour ce faire, on installe un filtre à l'extrémité d'un cathéter que l'on laisse en place et retire le moment venu. Mais le cathéter peut provoquer des adhérences, qui déchirent la paroi vasculaire lors du retrait de celui-ci. En plus, le cathéter mesurant une cinquantaine de centimètres devant rester partiellement à l'extérieur de l'organisme, est source d'infection. C'est pourquoi les malades munis d'un dispositif temporaire sont soumis en permanence à un traitement médicamenteux à base d'antibiotiques et à une hygiène draconienne.

Il serait donc souhaitable de pouvoir installer dans la veine cave inférieure et dans d'autres vaisseaux, un filtre parapluie de type définitif, c'est à dire du type où les brins métalliques sont terminées, à leur extrémité libre, par un crochet, mais qu'il soit cependant possible d'enlever.

Le document WO-00/16845 relève de l'art. 54 (3) CBE et divulgue un kit pour le retrait d'un filtre pour vaisseaux sanguins.

Or, après de longues recherches la demanderesse a découvert qu'un kit comprenant un cathéter et unetige terminée par des branches élastiques à crochets permettait d'agripper les filtres parapluie de type définitif et de resserrer leurs brins pour les décrocher de la paroi vasculaire sans la léser.

C'est pourquoi le présent brevet a pour objet un kit selon la revendication 1.

Il est ainsi possible de resserrer les branches installées à l'extrémité de la tige, en poussant la tige de les introduire dans le premier cathéter jusqu'à proximité du bout dudit premier cathéter, de faire progresser l'ensemble jusqu'à proximité de l'ogive du filtre et de faire progresser la tige pour faire sortir les branches et ainsi les laisser s'évaser. Il ne reste plus alors qu'à faire progresser à son tour le premier cathéter pour refermer les branches sur l'ogive et ainsi l'agripper, faire encore progresser le premier cathéter pour refermer les brins du filtre en les englobant dans celui-ci, puis tirer la tige pour extraire le filtre, éventuellement en même temps que le premier cathéter.

Le second cathéter, de diamètre apte à être intraduit dans le cathéter externe, permet d'installer dès l'origine la tige et ses branches élastiques resserrées dans ledit second cathéter, ce qui évite notamment d'avoir à resserrer les branches installées à l'extrémité de la tige au moment de l'utilisation. La tige préinstallée dans le second cathéter peut être conditionnée séparément avec ce dernier dans un second emballage stérile.

L'introduction dans des vaisseaux sanguins d'un cathéter creux, même émoussé, n'est pas facile à réaliser sans risques de lésions.

C'est pourquoi le présent brevet a aussi pour objet un kit ci-dessus, caractérisé en ce qu'il comprend en outre un troisième cathéter, de diamètre adapté à l'introduction dans le cathéter externe, dont l'extrémité avant est fermée et émoussée pour servir de dilatateur lors de l'introduction dans un vaisseau de l'ensemble constitué par le premier et ledit troisième cathéter.

Dans la mesure où le troisième cathéter doit servir de dilatateur, on comprend que sa longueur est supérieure à celle du premier cathéter et donc qu'il dépassera l'extrémité dudit premier cathéter lors de l'introduction dans les vaisseaux. Le diamètre externe du troisième cathéter est avantageusement ajusté au diamètre Interne du premier cathéter pour Juste glisser.

On utilise avantageusement le polyéthylène comme matériau pour réaliser les cathéters.

La tige et ses branches munies de crochets sera de préférence réalisée en acier inoxydable. Son éventuelle poignée pourra être par exemple réalisée elle aussi en polyéthylène.

La longueur des cathéters pourra aller par exemple de 20 à 100 cm, de préférence de 30 à 90 cm, notamment de 40 à 80 cm, tout particulièrement de 45 à 60 centimètres.

Le diamètre interne des cathéters pourra aller par exemple de 4,7 mm à 2,3 mm, de préférence de 4,3 à 2,7 mm, tout particulièrement de 3,3 à 2,7 mm pour le cathéter externe et par exemple de 4,0 mm à 1,67 mm, notamment 3,3 mm à 2 mm, particulièrement de 2,7 à 2 mm pour le second cathéter. On utilisera notamment un diamètre 9F pour le premier cathéter et un diamètre 7F pour le second cathéter. Rappelons que des diamètres de 9F et de 7F correspondent respectivement à 3,0 et 2,3 mm.

Dans des conditions préférentielles de mise en oeuvre du kit ci-dessus décrit, le troisième cathéter comprend au moins un dispositif détectable extracorporellement situé vers son extrémité avant. Ainsi, il est possible de suivre la progression de l'ensemble en direction du filtre.

Le dispositif détectable extracorporellement pourra être par exemple une bague notamment radio-opaque installée ou insérée sur un cathéter, de préférence en acier inoxydable, particulièrement en or, tout particulièrement en platine-iridium. Il peut aussi être un pointou latotalité d'un cathéter pourra être radio-opaque.

Dans d'autres conditions préférentielles de mise en oeuvre du kit ci-dessus décrit, le dispositif détectable extracorporellement est détectable par le même dispositif de détection que celui utilisable pour l'ogive du filtre. Ainsi un seul et même appareil de détection, par exemple de radioscopie, permet de localiser dans le corps le filtre et son ogive, et de suivre le cheminement des cathéters dans leur direction.

Le kit selon l'invention, dans sa version la plus élaborée mettant en jeu outre la tige deux cathéters creux et un cathéter dilatateur peut être utilisé comme suit, dans le cas d'une veine cave inférieure :

On ponctionne la veine jugulaire droite à l'aide d'une aiguille de ponction. On fait descendre un guide en forme de J recouvert de TEFLON^{®} à l'aide d'un raidisseur jusqu'à 5 cm au-dessus du filtre à extraire, en suivant sa progression par radioscopie. On retire l'aiguille de ponction. On fait glisser sur le guide en forme de J l'ensemble du premier et du troisième cathéters jusqu'à sa partie distale en suivant sa progression par radioscopie grâce à une bague radio-opaque. On retire en même temps le guide en J et le troisième cathéter sous radioscopie de face et surtout de profil. On installe une cale entre la poignée de la tige terminée par des branches élastiques à crochets et l'entrée du second cathéterpour éviter de repousser par inadvertance la tige et ouvrir en parapluie la pince à crochets. On introduit le second cathéter dans lequel est installée la tige en s'assurant de ne pas faire descendre celle-ci au-delà des 5 cm de garde prévue et en continuant à suivre par radioscopie toute la manipulation. On ôte alors la cale et on avance un peu la tige pour ouvrir la pince à crochets. Une fois les crochets récupérateurs ouverts, on descend lentement au-dessus de l'ogive du filtre à récupérer. On s'assure que les crochets se placent suffisamment en dessous de l'ogive. A ce moment de la procédure, on fait glisser lentement le premier cathéter sur les crochets à l'extrémité de la tige. On bloque en installant à nouveau la cale ou une cale un peu plus courte. Toujours sous radioscopie, on s'assure que les crochets de la pince sont bien refermés sous l'ogive du filtre et bien centrés. On continue à descendre lentement le premiercathéter au-delà de l'ogive du filtre, de façon à en replier les brins vers l'intérieur. On s'assure que les crochets du filtre sont bien décrochés de la paroi vasculaire. D'un mouvement lent de retrait, on retire la tige et le filtre qu'elle a accroché en s'assurant que le filtre a été entièrement attiré dans le second cathéter. On ressort l'ensemble du premier et du second cathéter avec la tige et le filtre. On assure alors l'hémostase au point de ponction.

L'invention sera mieux comprise si l'on se réfère aux dessins annexés sur lesquels
- la figure 1 représente une vue en élévation d'un premier cathéter, dit « externe » selon l'invention,
- la figure 2 représente une vue en élévation d'un second cathéter selon l'invention avec une tige en position pour agripper l'ogive d'un filtre.
- la figure 3 représente une vue en élévation d'un troisième cathéter selon l'invention servant de dilatateur,
- la figure 4 représente un filtre pour vaisseau sanguin du type parapluie.

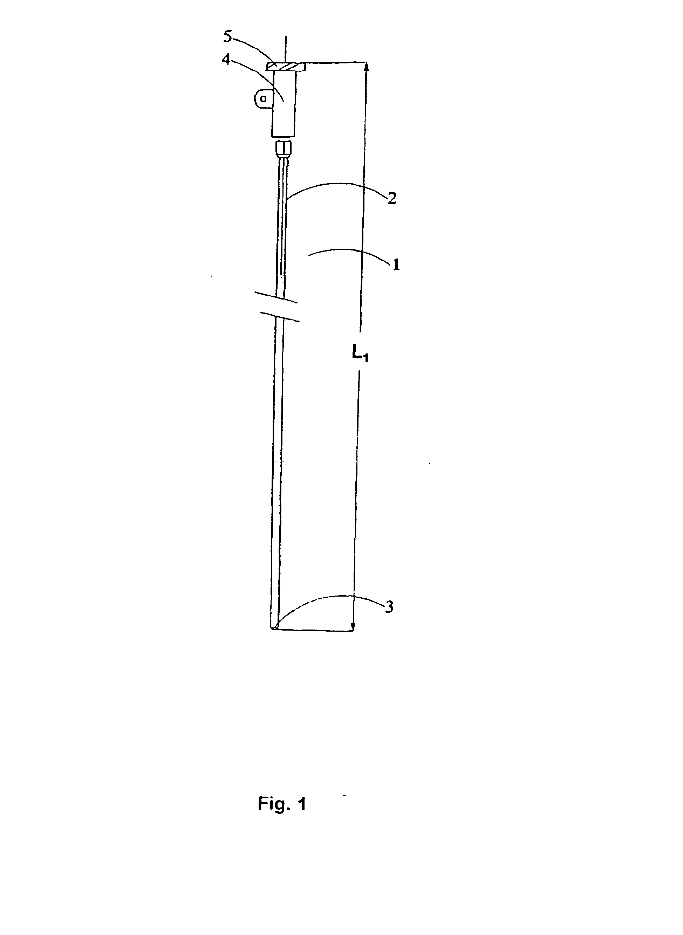

Sur la figure 1 on distingue un premier cathéter 1, dit « externe » constitué d'un tube creux 2 en polyéthylène de diamètre interne 9F (3,0 mm) muni d'une extrémité avant 3 émoussée. L'extrémité arrière est munie d'une partie élargie 4 pour en faciliter la manipulation, terminée par une collerette 5. L'extrémité avant 3 est si désiré munie d'une zone annulaire radio-opaque détectable extracorporellement. Sa longueur L₁ est de 550 mm.

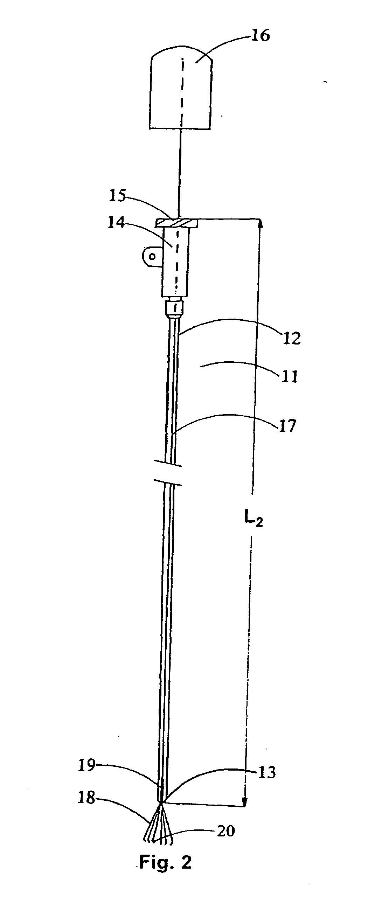

Sur la figure 2 on peut observer un second cathéter 11. Ce second cathéter a la même configuration générale que le premier cathéter. Il est donc muni d'une extrémité avant 13 émoussée. L'extrémité arrière est munie d'une partie élargie 14 terminée par une collerette 15. Sa longueur L₂ est de 553 mm etson diamètre interne 7F (2,3 mm) pour être inséré dans le premier cathéter 1.

Une tige est installée en position pour agripper l'ogive d'un filtre. Elle comprend une poignée de manipulation 16 en polyéthylène surmoulée sur la tige 17 proprement dite fabriquée en acier inoxydable. A l'autre extrémité se trouve une pluralité de branches élastiques 18 en acier inoxydable également, serties sur la tige 17 grâce à une gaine 19. Ces branches élastiques 18 comportent à leur extrémité des crochets 20 dirigés vers l'intérieur pour agripper l'ogive du filtre en se resserrant. Elles s'évasent à partir de la tige 17, et sont naturellement écartés les uns des autres si on n'exerce aucun effort sur elles. On comprend que si l'on fait progresser le premier cathéter 1 vers l'extrémité de la tige, on va refermer les branches 18 en rapprochant les crochets 20 les uns des autres. C'est précisément dans cette position branches resserrées que l'on peut introduire l'ensemble du second cathéter 11 et de la tige dans le premier cathéter 1.

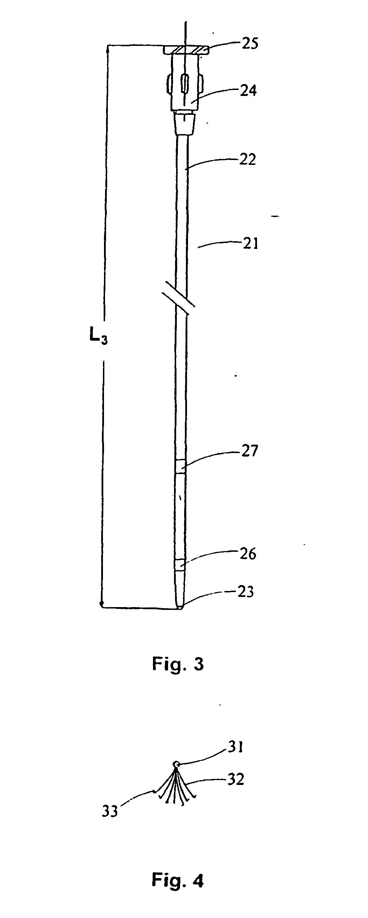

Sur la figure 3 on peut observer un troisième cathéter 21 servant de dilatateur. Ce troisième cathéter 21 a partiellement la même configuration générale que le premier et second cathéters. Il est donc muni d'une extrémité arrière munie d'une partie élargie 14 terminée par une collerette 25. Son extrémité avant 23 est émoussée mais a une forme légèrement conique et surtout est fermée. Sa longueur L₃ est de 605 mm et son diamètre interne 7F pour être inséré dans le premier cathéter 1. Ce troisième cathéter 21 est muni de deux zones annulaires radio-opaques détectables extracorporellement grâce aux rayons X, l'une 26 à proximité de l'extrémité avant émoussée et l'autre 27 à 560 mm de son extrémité arrière.

Dans un premier temps on installe le troisième cathéter 21 dilatateur dans le premier cathéter 1, dit « externe », introduit l'ensemble dans le système vasculaire jusqu'au filtre, dans un second temps on remplace le troisième cathéter 21 devenu inutile par le second cathéter 11 contenant la tige, agrippe le filtre, le prélève en retirant la tige, le second cathéter 11 et le premier cathéter 1.

Sur la figure 4 on peut observer un filtre apte à être prélevé dans le système vasculaire grâce à un kit selon l'invention. Il comprend une ogive 31 servant de manchon de retenue à une pluralité de brins élastiques 32 naturellement écartés les uns des autres et terminés en crochets 33 dirigés vers l'extérieur pour se bloquer contre et dans la paroi d'un vaisseau. L'extrémité de chaque brin constituant le crochet 33 forme un angle de 91 à 95°, voire plus par rapport au brin 32 pour être ôté sans dommage. Le rebord que forme l'ogive 31 du côté des brins 32 lui permet d'être accrochée par les crochets 20 de la tige.

## Revendications

1. Un kit pour le retrait d'un filtre pour vaisseau sanguin du type parapluie formé d'une ogive (31) servant de manchon de retenue à une pluralité de brins élastiques (32) naturellement écartés les uns des autres et terminés en crochets (33) dirigés vers l'extérieur pour se bloquer sur la paroi d'un vaisseau, **caractérisé en ce qu'**il comprend
- un premier cathéter (1), dit « externe »
- un second cathéter (11), de diamètre externe apte à être introduit dans le cathéter externe (1), et
- une tige apte à s'insérer dans le cathéter externe et comportant à une de ses extrémités une pluralité de branches élastiques (18) au nombre de 6 qui sont des brins s'évasant à partir de la tige, naturellement écartées les unes des autres et terminées en crochets (20) dirigés vers l'intérieur pour agripper l'ogive (31) du filtre en se resserrant, ladite tige ayant une longueur supérieure à celle du second cathéter (11) et, avant son utilisation pour agripper l'ogive (31) du filtre, étant installée dans le second cathéter (11) pour maintenir resserrées lesdites branches élastiques (18) de la tige.

2. Un kit selon la revendication 1, **caractérisé en ce qu'**il comprend en outre un troisième cathéter (21), de diamètre adapté à l'introduction dans le cathéter externe (1), dont l'extrémité avant (23) est fermée et émoussée pour servir de dilatateur lors de l'introduction dans un vaisseau de l'ensemble constitué par le premier (1) et ledit troisième (21) cathéter.

3. Un kit selon la revendication 2, **caractérisé en ce que** le troisième cathéter (21) comprend au moins un dispositif détectable extracorporellement (26) situé vers son extrémité avant.

4. Un kit selon la revendication 3, **caractérisé en ce que** le dispositif détectable extracorporellement (26) est détectable par le même dispositif de détection que celui utilisable pour l'ogive (31) du filtre.

5. Un kit selon l'une des revendications 1 à 4 **caractérisé en ce que** la longueur des cathéters va de 40 à 80 cm.

6. Un kit selon l'une des revendications 1 à 5 **caractérisé en ce que** le diamètre interne des cathéters va de 4,7 mm à 2,3 mm pour le cathéter externe et de 4,0 mm à 1,67 mm pour le second cathéter.

7. Un kit selon l'une des revendications 3 à 6 **caractérisé en ce que** le dispositif détectable extracorporellement comprend une bague par exemple radio-opaque.

## Patentansprüche

1. Kit zum Entfernen eines Blutgefäßfilters des Schirmtyps, der aus einer Spitze (31) gebildet ist, die 5 als Haltemuffe für mehrere elastische Fasern (32) dient, die voneinander auf natürliche Weise beabstandet sind und in Haken (33) enden, die nach außen gerichtet sind, um an der Wand eines Gefäßes zu blockieren, **dadurch gekennzeichnet, dass** es enthält:
- einen ersten, so genannten "externen" Katheter (1),
- einen zweiten Katheter (11) mit einem Durchmesser, der zulässt, dass er in den externen Katheter (1) eingeführt werden kann,
- einen Stift, der in den externen Katheter eingeführt werden kann und an einem seiner Enden mehrere elastische Zweige (18) aufweist, die zu sechst und Fasern sind, die sich ausgehend von dem Stift aufweiten, voneinander auf natürliche Weise beabstandet sind und in Haken (20) enden, die nach innen gerichtet sind, um die Spitze (31) des Filters zu ergreifen, indem sie sich zusammenziehen, wobei der Stift eine Länge besitzt, die größer als jene des zweiten Katheters (11) ist, und vor seiner Verwendung zum Ergreifen der Spitze (31) des Filters in dem zweiten Katheter (11) installiert ist, um die elastischen Zweige (18) des Stifts zusammen zu halten.

2. Kit nach Anspruch 1, **dadurch gekennzeichnet, dass** es außerdem einen dritten Katheter (21) enthält, dessen Durchmesser zulässt, dass er in den externen Katheter (1) eingeführt werden kann, dessen vorderes Ende (23) verschlossen und abgestumpft ist, um bei der Einführung in ein Gefäß der durch den ersten Katheter (1) und den dritten Katheter (21) gebildeten Gesamtheit als Ausdehner zu dienen.

3. Kit nach Anspruch 2, **dadurch gekennzeichnet, dass** der dritte Katheter (21) wenigstens eine von außerhalb des Körpers detektierbare Vorrichtung (26) aufweist, die sich an seinem vorderen Ende befindet.

4. Kit nach Anspruch 3, **dadurch gekennzeichnet, dass** die von außerhalb des Körpers detektierbare Vorrichtung (26) durch dieselbe Detektionsvorrichtung wie jene, die für die Spitze (31) des Filters verwendbar ist, detektiert werden kann.

5. Kit nach einem der Ansprüche 1 bis 4, **dadurch gekennzeichnet, dass** die Länge der Katheter im Bereich von 40 bis 80 cm liegt.

6. Kit nach einem der Ansprüche 1 bis 5, **dadurch gekennzeichnet, dass** der Innendurchmesser der Katheter für den externen Katheter im Bereich von 4,7 mm bis 2,3 mm und für den zweiten Katheter im Bereich von 4,0 mm bis 1,67 mm liegt.

7. Kit nach einem der Ansprüche 3 bis 6, **dadurch gekennzeichnet, dass** die von außerhalb des Körpers detektierbare Vorrichtung einen Ring, beispielsweise einen strahlungsundurchlässigen Ring, aufweist.

## Claims

1. A kit for the withdrawal of a blood vessel filter of the umbrella type formed by a bush (31) serving as a retaining sleeve for a number of flexible strands (32) which spread apart naturally and end in hooks (33) directed outwards so that they lock onto the wall of a vessel, **characterized in that** it comprises
- a first, so-called "external" catheter (1),
- a second catheter (11), with an outer diameter introducible into the external catheter (1),
- a stem insertable into the external catheter and has, at one of its ends, a plurality of flexible legs (18), the number of which is 6 and which open out from the stem, spreading apart naturally and ending in hooks (20) directed inwards for grasping the bush (31) of the filter as they close up back, said stem having a length that is greater than that of the second catheter (11) and, prior to its use for grasping the bush (31) of the filter, is installed inside the second catheter (11) so that said flexible legs (18) of the stem are kept closed up.

2. The kit according to Claim 1, **characterized in that** it additionally comprises a third catheter (21), of diameter suitable for introduction into the external catheter (1), the front end (23) of which is closed and chamfered for serving as a dilator during introduction of the assembly consisting of the first catheter (1) and said third catheter (21) into a vessel.

3. The kit according to Claim 2, **characterized in that** the third catheter (21) includes at least one extracorporeally detectable device (26) located near its front end.

4. The kit according to Claim 3, **characterized in that** the extracorporeally detectable device (26) is detectable by the same detecting device as the one usable for bush (31) of the filter.

5. The kit according to one of Claims 1 to 4, **characterized in that** the length of the catheters ranges from 40 to 80 cm,

6. The kit according to one of Claims 1 to 5, **characterized in that** the inside diameter of the catheters ranges from 4.7 mm to 2.3 mm for the external catheter and from 4.0 mm to 1.67 mm for the second catheter.

7. The kit according to one of Claims 3 to 6, **characterized in that** the extracorporeally detectable device comprises a ring, for example radiopaque.
